# EUROPEAN PATENT APPLICATION

(11) **EP 4 442 322 A1**
(43) Date of publication of application: **09.10.2024**
(21) Application number: 23167117.3
(22) Date of filing: 06.04.2023
(51) Int. Cl.: A61P 37/06, A61K 39/00, C07K 16/28

(54) **LIVER-SPECIFIC CAR FOR USE AGAINST IMMUNOLOGIC REJECTION**

(71) Applicant: Medizinische Hochschule Hannover, 30625 Hannover (DE); Technische Universität Braunschweig, 38106 Braunschweig (DE)
(72) Inventor: Hardtke-Wolenski, Matthias, Hannover (DE); Jäckel, Elmar, Hannover (DE); Hust, Michael, Braunschweig (DE)
(74) Representative: Taruttis, Stefan Georg

(57) **Abstract**

The present invention relates to a CAR and to regulatory T-cells (Treg) expressing the CAR, especially for use in the treatment, especially for suppression, of adverse immune reactions against liver tissue, e.g. for use in the treatment of host-versus-graft disease (HvG) against a liver transplant, for use in the treatment of an autoimmune disease directed against liver tissue, or for use in the treatment of inflammatory liver disorder. The invention provides a chimeric antigen receptor (CAR) that comprises an scFv portion, which is specific for the asialoglycoprotein receptor (ASGPR).

## Description

The present invention relates to a CAR and to regulatory T-cells (Treg) expressing the CAR, especially for use in the treatment, especially for suppression, of adverse immune reactions against liver tissue, e.g. for use in the treatment of host-versus-graft disease (HvG) against a liver transplant, for use in the treatment of an autoimmune disease directed against liver tissue, or for use in the treatment of inflammatory liver disorder. Treg of the invention that express the CAR induce immune tolerance specifically in liver tissue by liver-localized suppression of adverse immune reaction. Further, Treg of the invention that express the CAR migrate to the liver, allowing their use in the treatment by administration to a patient at a site spaced from the liver, e.g. administration by systemic injection, e.g. intra venous administration.

The Treg of the invention that express the CAR are suitable for use in suppressing adverse immune reactions directed against liver tissue, e.g. for use in the treatment of HvG, without specificity for a foreign antigen, especially without specificity for an immunologically incompatible HLA. Therefore, the Treg expressing the CAR of the invention are independent of a foreign antigen that is present in the liver but they are suitable for inducing immune tolerance that is essentially limited to liver tissue and depends of presence of liver cells, especially depends on the presence of hepatocytes only.

An advantage of embodiments of the CAR of the invention and of Treg expressing the CAR is that the CAR is liver-specifically effective both in humans and in rodents, e.g. mice and rats.

### State of the art

WO 2021/038249 A1 without any evidence for its therapeutic effect claims use in HvG of a Treg expressing a CAR specific for asialoglycoprotein receptor. Due to this lack of disclosure, WO 2021/038249 A1 does not qualify as prior art.

US 2018/0201902 A1 as well as Chen et al., Cancer Immunol. Immunother. 2017, 475-489 for use in liver cancer therapy describe T-cells expressing both a CAR specific for GPC3 and a CAR specific for asialoglycoprotein receptor 1.

Rigopoulou et al., Autoimmunity Reviews 2012, 260-269 review asialoglycoprotein receptor (ASGPR) as a target autoantigen in liver autoimmunity, presence of anti-ASGPR autoantibodies during the course of autoimmunity disease and immunosuppressive treatments.

### Object of the invention

It is an object of the invention to provide a liver-specific immune suppression, which is limited to liver tissue and is preferably independent from presence of a heterologous antigen.

### Description of the invention

The invention achieves the object by the features of the claims, and especially provides a chimeric antigen receptor (CAR) that from N-terminus to C-terminus comprises or consists of an scFv portion, which is specific for the asialoglycoprotein receptor (ASGPR), especially for the human and/or for the murine and rat ASGPR, preferably a hinge region, e.g. a hinge comprising IgG1 - IgG1 CH2 - IgG1 CH3 hinge, a transmembrane domain (TM), e.g. a CD4 TM or a CD4a TM, and intracellular signaling domains, e.g. a CD28 signaling domain and a CD3zeta signaling domain, wherein preferably the scFv portion has a pair of complementary domain regions 3 (CDR3) of the heavy and light chain, the pairs of CDR3 having an amino acid sequence which comprise or consist of
amino acids No. 97..113 of SEQ ID NO: 28 and amino acids No. 89..97 of SEQ ID NO: 29, or which comprise or consist of amino acids No. 99..109 of SEQ ID NO: 30 and amino acids No. 91..101 of SEQ ID NO: 31,
or which comprise or consist of amino acids No. 97..108 of SEQ ID NO: 32 and amino acids No. 92..100 of SEQ ID NO: 33,
or which comprise or consist of amino acids No. 97..108 of SEQ ID NO: 34 and amino acids No. 92..100 of SEQ ID NO: 35,
or which comprise or consist of amino acids No. 97..107 of SEQ ID NO: 36 and amino acids No. 89..97 of SEQ ID NO: 37.

Preferably, the scFv has a variable heavy chain (VH) comprising or consisting of amino acid sequence
QVQLVQSGAEVKKPGASVKVSCKASGYTFTSYYMHWVRQAPGQGPEWMGIINPSG GSTSYAQKFQGRVTMTRDTSTSTVYMELSSLRSEDTAVYYCARDAVGLRERYYYGM DVWGQGTTVTVSS (SEQ ID NO: 28, VH of G3) and a variable light chain (VL) comprising or consisting of amino acid sequence 
DIVMTQSPSSLSASVGDRVTITCRASQSISSYLNWYQQKPGKAPKLLIYAASSLQSGVP
SRFSGSGSGTDFTLTISSLQPEDFATYYCQQSYSTPFTFGGGTKVEIK (SEQ ID NO: 29,
VL of G3),
or a VH comprising or consisting of amino acid sequence
QIQLVQ SGGGLVQPGGSLRLSCAGSGYTF SDHYMDW VRQAPGKGLEW VGRIRYKA NSYSTEYAASVKGRFTISRDDSRNSLFLQMNSLKTEDTAVYYCARIRDRYYFDYWGQ GALVTVSS (SEQ ID NO: 30, VH of C11) and a variable light chain (VL) comprising or consisting of amino acid sequence 
QSVLTQPPSVSGAPGQRVTISCTGSSSNVGAGYDVHWYQQLPGAAPKLLIYGNSNRP
SGVPDRFSGSKSGTSASLAISGLQSEDEADYYCAAWDDSLNGVVFGGGTKLTVL
(SEQ ID NO: 31, VL of C11),
or a VH comprising or consisting of amino acid sequence
QVQLVQSGAEVKKPGASVKVSCKASGYTFTSYGISWVRQAPGQGLEWMGWISAYN GNTNYAQKLQGRVTMTTDTSTSTAYMELRSLRSDDTAVYYCARGYSYGDDFDYWG QGTLVTVSS (SEQ ID NO: 32, VH of E12) and a variable light chain (VL) comprising or consisting of amino acid sequence 
NFMLTQPHSVSESPGKTVILSCTGTGGSIASNYVQWYQQRPGSAPTTVIYKDNERPSG
VPDRFSGAIDSSSNSASLTISGLRTEDEADYYCQSYGSGNVVFGGGTKLTVL
(SEQ ID NO: 33, VL of E12),
or a VH comprising or consisting of amino acid sequence
QVTLKESGGGLVQPGGSLRLSCAASGFTFSSYAMSWVRQAPGKGLEWVSAISGSGGS TYYADSVKGRFTISRDNSKNTLYLQMNSLRAEDTAVYYCAKDLKQWLEDYWGQGT LVTVSS (SEQ ID NO: 34, VH of C3) and a variable light chain (VL) comprising or consisting of amino acid sequence 
DVVMTQSPSTLSASVGDRVTITCRASQSIGPWLAWYQQKPGEAPKLLLYKASILEGG
VPSRFSGRGSGTEFTLTISSLQPDDFATYYCQQYNTFGPTFGQGTKLEIK
(SEQ ID NO: 35, VL of C3),
or a VH comprising or consisting of amino acid sequence
QVQLVQ SGAEVKKPGAS VKV SCKASGYTFTGYYMHW VRQAPGQGLEWMGWINPN SGGTNYAQKFQGWVTMTRDTSISTAYMELSRLRSDDTAVYYCARKAVAADYFDYW GQGTLVTVSS (SEQ ID NO: 36, VH of B10) and a variable light chain (VL) comprising or consisting of amino acid sequence

NFMLTQPHSVSASPGKTVTISCTGSGGSIASHYVQWYQQRPGSAPTSVIYEDDQRPSG
VPDRFSGSIDSSSNSASLTISGLKTEDEADYYCQSYDSSHQVFGGGTKLTVL
(SEQ ID NO: 37, VL of B10).

For localization of the CAR, its amino acid sequence preferably comprises at its N-terminus a signal peptide directing the CAR for transmembrane localization.

Generally preferred, the signal peptide which is arranged N-terminally of the scFv and the domains of the CAR which are arranged C-terminally of the scFv, e.g. a hinge, a transmembrane domain and signaling domains have sequences of human origin for use in the treatment of humans, especially in order to avoid immune reactions against these domains. Herein, domains or sections of a CAR having an amino acid sequence of human origin can be identified by a "h", and domains or sections having a murine amino acid sequence can be identified by an "m". For use in rodents, e.g. mice or rats, the signal peptide and the domains of the CAR which are arranged C-terminally of the scFv can have sequences of murine origin or, less preferred, of human origin.

Optionally, the CAR is expressed as a fusion protein with FOXP3, which for use in the treatment of a human preferably has the human amino acid sequence of FOXP3 or for use in an experimental has the autologous amino acid sequence, e.g. for use in mice has the murine amino acid sequence (Foxp3). Generally, the fusion protein from N-terminus to C-terminus may comprise or consist of the CAR and FOXP3, separated by a protease site, e.g. P2A, or consist of the CAR and optionally in addition deltaLNGFR. Suitably, the FOXP3 polypeptide encoded by a nucleic acid molecule, construct or vector as described herein may comprise or consist of the polypeptide sequence of a human FOXP3, such as UniProtKB accession Q9BZS1, or a functional fragment or variant thereof.

Optionally, the CAR is expressed from an expression cassette which, under the control of an IRES, encodes a peptide label, which preferably for use in humans is deltaLNGFR (SEQ ID NO: 39, encoded by SEQ ID NO: 38), and for use in mice is Thy 1.1 (also termed CD90.1). The peptide deltaLNGFR (SEQ ID NO: 39), in addition to presenting a surface-bound label which is not immunogenic in humans and which can be detected by an antibody, e.g. a labelled antibody, deltaLNGFR also provides a peptide label suitable for specifically triggering the deletion of cells that express the CAR, e.g. using an anti-deltaLNGFR-antibody. Preferably, Treg are genetically manipulated for expression of the CAR in combination with FOXP3, e.g. expression from one common nucleic acid, preferably from one common expression cassette, with an IRES arranged between the coding sequence encoding the CAR, a protease site, and optionally FOXP3, and the coding sequence (SEQ ID NO: 38) encoding deltaLNGFR. Genetic manipulation can be by transduction of T cells, preferably of Treg, by a viral particle, preferably a retroviral particle, especially an α-retroviral or a γ-retroviral particle, the viral particle containing an expression cassette between its 5'LTR and its 3'LTR.

In embodiments of Treg that in addition to the CAR, preferably also Foxp3, also express Thy 1.1, deletion of these Treg can be triggered specifically by an anti-Thy 1.1-antibody.

In this embodiment, the Treg are suitable for use in the treatment of adverse immune reactions directed against liver tissue with the additional feature of terminating the activity of the Treg, e.g. deleting the Treg, by using an antibody specific for the marker peptide, herein represented by deltaLNGFR for use in humans, or Thy 1.1 for use in mice, which represents a suicide moiety for binding of an antibody.

The safety switch polypeptide provides a cell in or on which it is expressed with a suicide moiety. This is useful as a safety mechanism which allows a cell which has been administered to a subject to be deleted should the need arise, or indeed more generally, according to desire or need, for example once a cell has performed or completed its therapeutic effect.

A suicide moiety possesses an inducible capacity to lead to cellular death, or more generally to elimination or deletion of a cell. An example of a suicide moiety is a suicide protein, encoded by a suicide gene, which may be expressed in or on a cell alongside a desired transgene, in this case the CAR, which when expressed allows the cell to be deleted to turn off expression of the transgene (CAR). A suicide moiety herein is a suicide polypeptide that is a polypeptide that under permissive conditions, namely conditions that are induced or turned on, is able to cause the cell to be deleted.

The suicide moiety may be a polypeptide, or amino acid sequence, which may be activated to perform a cell-deleting activity by an activating agent which is administered to the subject, or which is active to perform a cell-deleting activity in the presence of a substrate which may be administered to a subject. In a particular embodiment, the suicide moiety may represent a target for a separate cell-deleting agent which is administered to the subject. By binding to the suicide moiety, the cell-deleting agent may be targeted to the cell to be deleted. In particular, the suicide moiety may be recognised by an antibody, and binding of the antibody to the safety switch polypeptide, when expressed on the surface of a cell, causes the cell to be eliminated, or deleted.

The suicide moiety may be HSV-TK or iCasp9. However, it is preferred for the suicide moiety to be, or to comprise, an epitope which is recognised by a cell-deleting antibody or other binding molecule capable of eliciting deletion of the cell. In such an embodiment, the safety switch polypeptide is expressed on the surface of a cell.

The term "delete" as used herein in the context of cell deletion is synonymous with "remove" or "ablate" or "eliminate". The term is used to encompass cell killing, or inhibition of cell proliferation, such that the number of cells in the subject may be reduced. 100% complete removal may be desirable but may not necessarily be achieved. Reducing the number of cells, or inhibiting their proliferation, in the subject may be sufficient to have a beneficial effect.

In particular, the suicide moiety may be a CD20 epitope which is recognised by the antibody Rituximab. Thus, in the safety switch polypeptide the suicide moiety may comprise a minimal epitope based on the epitope from CD20 that is recognised by the antibody Rituximab. Biosimilars for Rituximab are available and may be used. A person of skill in the art is readily able to use routine methods to prepare an antibody having the binding specificity of Rituximab using the available amino acid sequences therefor.

CAR-cells specific for ASGPR, which also express a safety switch polypeptide comprising this sequence can be selectively killed using the antibody Rituximab, or an antibody having the binding specificity of Rituximab. The safety switch polypeptide is expressed on the cell surface and when the expressed polypeptide is exposed to or contacted with Rituximab, or an antibody with the same binding specificity, death of the cell ensues.

Thus, Rituximab, or an antibody having the binding specificity thereof, may be provided for use in adoptive cell transfer (ACT) in combination with a cell of the invention. The cell or nucleic acid or vector or construct for production of the cell and the Rituximab or equivalent antibody may be provided in a kit, or as a combination product.

For example, the suicide constructs of WO2013/153391 or WO2021/239812 may be used in a cell or cell population (e.g., Treg or Treg population) as described herein.

It was found that the specificity of the scFv portion for ASGPR essentially depends on the CDR3 domains of the heavy chain and of the light chain of the scFv, and that this specificity can be maintained also in combination with other CDR1 and CDR2 domains that originate from a paratope, e.g. an scFv, which is not specific for ASGPR.

Generally preferred, the Treg are immunologically compatible to the recipient, and preferably the Treg originate from T cells originating from the recipient, i.e. the Treg are derived from autologous T cells, wherein they are derived by genetic manipulation to express the CAR, preferably in combination with FOXP3, and optionally express the peptide label, which preferably is CD20 or deltaLNGFR for use in humans, or Thy 1.1 for use in mice.

The signal peptide can e.g. have the amino acid sequence MDFQVQIFSFLLISASVIMSRT (SEQ ID NO: 1, murine) or MWWRLWWLLLLLLLLWPMVWA (SEQ ID NO: 2, murine), preferably MWWRLWWLLLLLLLLWPMVW (SEQ ID NO: 3, human), or MDFQVQIFSFLLISASVIMSR (SEQ ID NO: 4, human).

The hinge domain, which connects the scFv portion with the transmembrane domain, can e.g. have an amino acid sequence comprising or consisting of the IgG1 hinge, the IgG1 CH2 hinge and the IgG1 CH3 hinge, the hinge region of CD28, of Cd8α, of CD4, of CD7, of CH2CH3, of an immunoglobulin, or a part or variant thereof, preferably the CD8α hinge domain or CH2CH3 hinge domain, all of human origin or all of murine origin, e.g. VPRDGGCKPCICT VPEVS S VFIFPPKPKD VLTITLTPK VTC VVVDISKDDPEVQF S WF V DDVEVHTAQTQPREEQFNSTFRSVSELPIMHQDWLNGKEFKCRVNSAAFPAPIEKTIS KTKGRPKAPQVCTIPPPKEQMAKDKVSLTCMITDFFPEDITVEWQWNGQPAENYKNT QPIMDTDGSYFVYSKLNVQKSNWEAGNTFTCSVLHEGLHNHHTEKSLSHS PG (SEQ ID NO: 5, murine) which consists of the mIgG1 hinge, the mIgG1 CH2 hinge and the mIgG1 CH3 hinge, or the CD8 hinge of human origin or of murine origin, e.g. FSSVVPVLQKVNSTTTKPVLRTPSPVHPTGTSQPQRPEDCRPRGSVKGTGLDFACDIY (SEQ ID NO: 6, murine), or e.g. FVPVFLPAKPTTTPAPRPPTPAPTIASQPLSLRPEASRPAAGGAVHTRGLDFADIYIWAP LAGTCGVLLLSLVITLYCNHRQL (SEQ ID NO: 7, human) consisting of the hCD8 hinge and the hCD28 transmembrane domain (hCD8 TM), or the deltaFc Ig, which in the CAR has the functions of a hinge domain and a transmembrane domain, e.g. having the amino acid sequence

PAEPKSPDKTHTCPPCPAPPVAGPSVFLFPPKPKDTLMIARTPEVTCVVVDVSHEDPEV
KFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSV
MHEALHNHYTQKSLSLSPGKK (SEQ ID NO: 8, human).

A transmembrane domain may be selected from the transmembrane domains of CD28, of ICOS, of CD8α, of CD4, of CD134 (OX40), of CD137 (4-1BB), of CD3 zeta, of CD45, of CD9, of CD16, of CD22, of CD33, of CD64, of CD80, of CD86, of CD154, of CH2CH3, or a part or variant thereof. Preferably, the CAR comprises a transmembrane domain of CD8α or of CH2CH3. As examples, the transmembrane domain (TM) may be the CD4 TM, e.g. VFLACVLGGSFGFLGFLGLCILCCV (SEQ ID NO: 9, murine) or its human equivalent, or the CD8a transmembrane domain CD8a TM, e.g. IWAPLAGICVALLLSLIITLICYHR (SEQ ID NO: 10, murine) or its human equivalent.

Preferably, the intracellular signaling domains comprise or consist of, from N-terminus to C-terminus, the CD28 signaling domain (CD28 ICD) and the CD3zeta signaling domain (CD3z ICD). The CD28 ICD can e.g. have the amino acid sequence NSRRNRLLQSDYMNMTPRRPGLTRKPYQPYAPARDFAAYRPL (SEQ ID NO: 11, murine) and the CD3z ICD can e.g. have the amino acid sequence RAKF SRSAETAANLQDPNQLYNELNLGRREEYDVLEKKRARDPEMGGKQQRRRNPQ EGVYNALQKDKMAEAYSEIGTKGERRRGKGHDGLYQGLSTATKDTYDA LHMQTLAPR (SEQ ID NO: 12, murine), or the CD28 ICD can e.g. have the amino acid sequence WTNSRRNRLLQSDYMNMTPRRPGLTRKPYQPYAPARDFAAYRP (SEQ ID NO: 13, murine), the CD3z ICD can e.g. have the amino acid sequence

RAKFSRSAETAANLQDPNQLYNELNLGRREEYDVLEKKRARDPEMGGKQQRRRNPQ
EGVYNALQKDKMAEAYSEIGTKGERRRGKGHDGLYQGLSTATKDTYDALHMQTLA
PR (SEQ ID NO: 14, murine).

The human CD28 ICD can e.g. have the amino acid sequence
WVRSKRSRLLHSDYMNMTPRRPGPTRKHYQPYAPPRDFAAYRS (SEQ ID NO: 15, human), the human CD3z can have the amino acid sequence
RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDA LHMQALPPRSSR (SEQ ID NO: 16, human), or the human CD28 ICD can have the amino acid sequence
DPKFWVLVVVGGVLACYSLLVTVAFIIFWVRSKRSRLLHSDYMNMTPRRPGPTRKH YQAYAAARDFAAYRSL (SEQ ID NO: 17, human), and the human CD28 ICD can have the amino acid sequence

RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDA
LHMQALPPR (SEQ ID NO: 18, human).

The CAR preferably has an intracellular domain (ICD) comprising or consisting of one or more intracellular signaling domains selected from the group consisting of the CD3zeta signaling domain or any of its homologs, the signaling domain of a CD3 polypeptide, of a syk family tyrosine kinase, of a src family tyrosine kinase, of CD2, of CD5, and of CD8, or a signaling domain of a part or variant thereof. Preferably, the CAR comprises the CD3 zeta signaling domain. Generally preferred, the CAR comprises or consists of a human signal peptide, an scFv, human a CD28 hinge and a human CD28 transmembrane domain, a human CD28 ICD and a human CD3z ICD, or the CAR comprises or consists of a human signal peptide, an scFv, a human delta Fc Ig as a hinge and transmembrane domain, a human CD28 ICD and a human CD3z ICD, especially for use in the treatment of adverse immune reactions directed against the liver in a human.

The CAR may comprise one or more co-stimulatory domains. The one or more co-stimulatory domains may be selected from the intracellular domains of CD28, of ICOS, of CD134 (OX40), of CD137 (4-1BB), of CD27, or of TNFRSF25, or of a part or variant thereof. Preferably, the CAR may comprise a CD28 co-stimulatory domain. The one or more co-stimulatory domain can be arranged C-terminally to the transmembrane domain.

The CAR, especially the endodomains of the CAR which are arranged C-terminally to the transmembrane domain, may comprise one or more intracellular signaling domains selected from the group consisting of the CD3 zeta signaling domain or any of its homologs, a CD3 polypeptide signaling domain, a syk family tyrosine kinase signaling domain, a src family tyrosine kinase signaling domain, CD2 signaling domain, CD5 signaling domain, and CD8 signaling domain, or a part or variant thereof. Preferably, the CAR may comprise the CD3zeta signaling domain.

The CAR may comprise one or more co-stimulatory domains, e.g. arranged C-terminally to the transmembrane domain of the CAR. The one or more co-stimulatory domains may be selected from the intracellular domains of CD28, of ICOS, of CD134 (OX40), of CD137 (4-1BB), of CD27, or of TNFRSF25, or a part or variant thereof. Preferably, the CAR may comprise a CD28 co-stimulatory domain.

The CAR preferably comprises or consists of a signal peptide, an scFv, a hinge comprising Ig G1 hinge, Ig G1 CH2 hinge and Ig G1 CH3 hinge, a CD4 transmembrane domain, a CD28 ICD and a CD3zeta ICD, or the CAR comprises or consists of a signal peptide, an scFv, a CD8 hinge, a CD8a transmembrane domain, a CD28 ICD and a CD3z ICD, for use in humans in each case preferably all of human origin, for use in mice preferably all of murine origin. The CAR is preferably encoded as a fusion protein, in which C-terminally to the CAR, a protease site, e.g. P2A, and FOXP3 are linked. The P2A protease site can have the amino acid sequence ATNFSLLKQAGDVEENPGP (SEQ ID NO: 19), FOXP3 can have the amino acid sequence

MPNPRPAKPMAPSLALGPSPGVLPSWKTAPKGSELLGTRGSGGPFQGRDLRSGAHTS
SSLNPLPPSQLQLPTVPLVMVAPSGARLGPSPHLQALLQDRPHFMHQL
STVDAHAQTPVLQVRPLDNPAMISLPPPSAATGVFSLKARPGLPPGINVASLEWVSRE
PALLCTFPRSGTPRKDSNLLAAPQGSYPLLANGVCKWPGCEKVFEEPEE
FLKHCQADHLLDEKGKAQCLLQREVVQSLEQQLELEKEKLGAMQAHLAGKMALAK
APSVASMDKSSCCIVATSTQGSVLPAWSAPREAPDGGLFAVRRHLWGSH
GNSSFPEFFHNMDYFKYHNMRPPFTYATLIRWAILEAPERQRTLNEIYHWFTRMFAY
FRNHPATWKNAIRHNLSLHKCFVRVESEKGAVWTVDEFEFRKKRSQRP
NKCSNPCP (SEQ ID NO: 20, murine) or

ATNFSLLKQAGDVEENPGPSMPNPRPAKPMAPSLALGPSPGVLPSWKTAPKGSELLG
TRGSGGPFQGRDLRSGAHTSSSLNPLPPSQLQLPTVPLVMVAPSGARLGPSPHLQALL
QDRPHFMHQLSTVDAHAQTPVLQVRPLDNPAMISLPPPSAATGVFSLKARPGLPPGI
NVASLEWVSREPALLCTFPRSGTPRKDSNLLAAPQGSYPLLANGVCKWPGCEKVFEE
PEEFLKHCQADHLLDEKGKAQCLLQREVVQSLEQQLELEKEKLGAMQAHLAGKMA
LAKAPSVASMDKSSCCIVATSTQGSVLPAWSAPREAPDGGLFAVRRHLWGSHGNSSF
PEFFHNMDYFKYHNMRPPFTYATLIRWAILEAPERQRTLNEIYHWFTRMFAYFRNHP
ATWKNAIRHNLSLHKCFVRVESEKGAVWTVDEFEFRKKRSQRPNKCSNPCP (SEQ ID
NO: 21, murine).

Preferably FOXP3 (human) can have an amino acid sequence comprising or consisting of SEQ ID NO: 22.

The optional marker deltaLNGFR or CD20 for use in humans, or Thy 1.1 for use in mice, can e.g. be encoded by a nucleic acid sequence that is connected in 3' to the nucleic acid sequence encoding the CAR or preferably a fusion protein of the CAR and FOXP3 by an interstitial IRES. The expression of FOXP3 in combination with expression of the CAR maintains the suppressor activity of regulatory T cells (Treg).

The CAR is preferably expressed in regulatory T cells, which are characterized by expressing the markers CD4⁺CD25⁺CD127^{low}. Preferably, the regulatory T cells are immunologically compatible with the human to be treated, more preferably the regulatory T cells originate from the human to be treated who, after genetic manipulation of the T cells such that they express the CAR, can receive the T cells expressing the CAR.

The scFv domain of the CAR molecules of the invention were generated by phage display using a human naive phage display library HAL9/10. A cell panning approach was performed by panning against recombinant murine or human ASGPR. The phage display library or binding scFv of consecutive panning round were incubated with either murine or human ASGPR, immobilized for selecting phage displaying scFv. From eluted phage, the scFv were amplified and used for a next panning round. Panning was conducted for 3 consecutive rounds. After the last panning round, individual scFv were initially screened first for recognition of ASGPR. For screening, soluble scFv, provided with a His-Tag and a Myc-Tag were produced in E.coli and used to stain murine and human liver sections, and secondary staining was performed by PE staining the His-Tag of soluble scFv. Cells were measured in flow cytometry for PE (scFv binding).

The scFv that were identified as being soluble were used in liver section staining.

Cryo IHC was performed to prove islet recognition by selected scFv molecules in liver tissues. For this, fresh livers of C57Bl/6 mice were embedded in Tissue-Tek^{®} Cryomold^{®} and quick frozen in liquid nitrogen. 5 µm sections were prepared in a cryostat-microtome. Autofluorescence of sections was quenched by incubation in Glycin buffer. Blocking was performed by incubation in 2% BSA. Consecutively, sections were stained with rabbit α-Myc-Tag antibody, recognising the Myc Tag of soluble scFvs. Tertiary staining was performed by incubation with FITC-labelled goat α-rabbit antibody. Sections were embedded with Fluoromount-G^{™} Mounting Medium containing DAPI and analysed by fluorescence microscopy.

Cloning of murine and human scFvs into murine and human retroviral vectors, respectively, was performed to generate CARs with ASGPR specificity. The CARs either had a murine CD8α hinge and transmembrane domain (short hinge) or a murine IgG hinge domain and CD4 transmembrane domain (long hinge). The sequence of the CD8α hinge is shown in SEQ ID NO: 6. Retroviral vectors containing LTR flanked 2nd generation CAR scaffolds were used for cloning. Cloning was performed by digestion of vectors with Ncol/Notl restriction enzymes and subsequent ligation of the scFv encoding nucleic acid sequence into the CAR backbone. Murine CAR backbones contained an additional Foxp3 expression cassette allowing converted Tregs (cTreg) generation out of CD4+ T cells but lacked such a cassette for generation of CAR Teffs or natural Tregs (nTregs). Vectors also contained Thy1.1.

### (CD90.1) as a CAR expression marker.

Production of recombinant CAR expressing retroviral virus and transduction of T cells and cell lines:
Retroviral particles allowing for CAR transduction of T cells were produced in HEK293T cells. They were K73 ecotropic-pseudotyped for murine constructs and VSV-G pantropic-pseudotyped for human constructs, respectively. For CAR transduction, CD4+ cells were enriched from murine spleenocytes by magnetic bead separation and activated by addition of anti-CD3/anti-CD28 beads for 2 days, followed by spin-transduction with virus particles and protaminsulfate and then underwent continued cultivation for 1 - 4 days. For a CAR activation assay, NFAT-GFP reporter murine T cell hybridoma cells were transduced in an analogous manner.

Hybridoma NFAT activation assay:
Murine T cell hybridoma cell line reporting NFAT activation by GFP expression were transduced with CAR vector as described above. In parallel, hepatocytes were purified that all express the target antigen ASGPR. After 48 h both cells of the cell line and hepatocytes were cultivated for 24 h to allow for CAR activation. In a parallel approach, ASGPR peptide (human or murine) that was directly coated on wells of a microtiter plate was used to stimulate CAR transduced hybridoma cells. Wells that were directly coated with collagenase protein and untreated wells served as controls. Cells were stained with anti-Fab antibody binding to the CAR domains for detecting CAR expression. The level of CAR activation reported by GFP and CAR expression was measured by flow cytometry. Herein, this T cell hybridoma cell line is also referred to as the reporter cell line or as reporter cells, which is used in the examples, unless indicated otherwise.

The invention is now described in greater detail by way of examples and with reference to the figures, which show in
- Fig. 1 a schematic arrangement of a nucleic acid coding sequence for domains of a fusion protein of the CAR (mIg) and FOXP3 with the coding sequence for Thy 1.1 connected by an IRES,
- Fig. 2 a schematic arrangement of a nucleic acid coding sequence for domains of a fusion protein of the CAR (mCD8) and FOXP3 with the coding sequence for Thy 1.1 connected by an IRES,
- Fig. 3 a schematic arrangement of a nucleic acid coding sequence for domains of a fusion protein of the CAR (hCD8) and FOXP3 with the coding sequence for an exemplary marker protein (dLNGFR) connected by an IRES,
- Fig. 4 a schematic arrangement of a nucleic acid coding sequence for domains of a fusion protein of the CAR (hIg) with the coding sequence for an exemplary marker protein (dLNGFR) connected by an IRES,
- Fig. 5A shows flow cytometry results for control Fibroblast cell line SC1, and Fig. 5B-F show flow cytometry results for SC1 expressing a CAR of the invention,
- Fig. 6A shows flow cytometry results for activation of control T cells,
- Fig. 6D shows flow cytometry results for activation of T cells expressing the CAR but without stimulation,

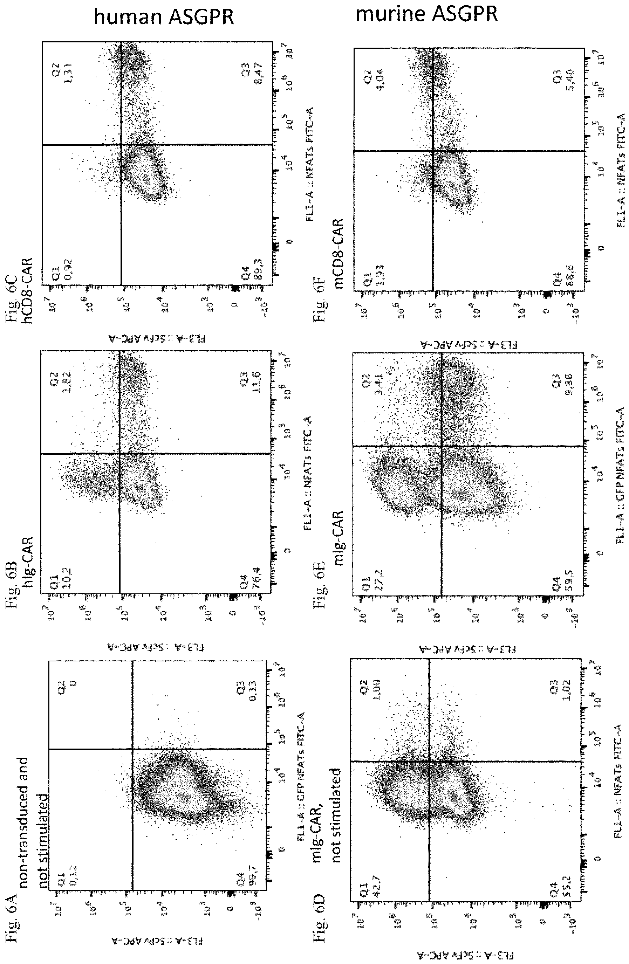

- Fig. 6B, Fig. 6C, Fig. 6E and Fig 6F show flow cytometry results for T cells expressing a CAR of the invention with stimulation,

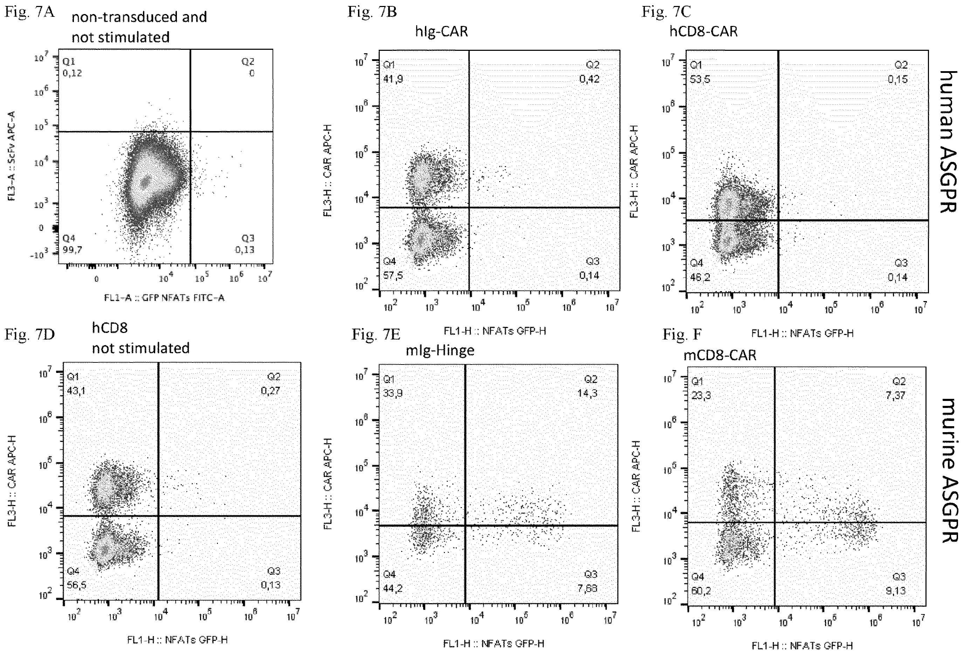

- Fig. 7A shows flow cytometry results for control Treg, and Fig. 7B-E show flow cytometry results for Treg expressing a CAR of the invention,

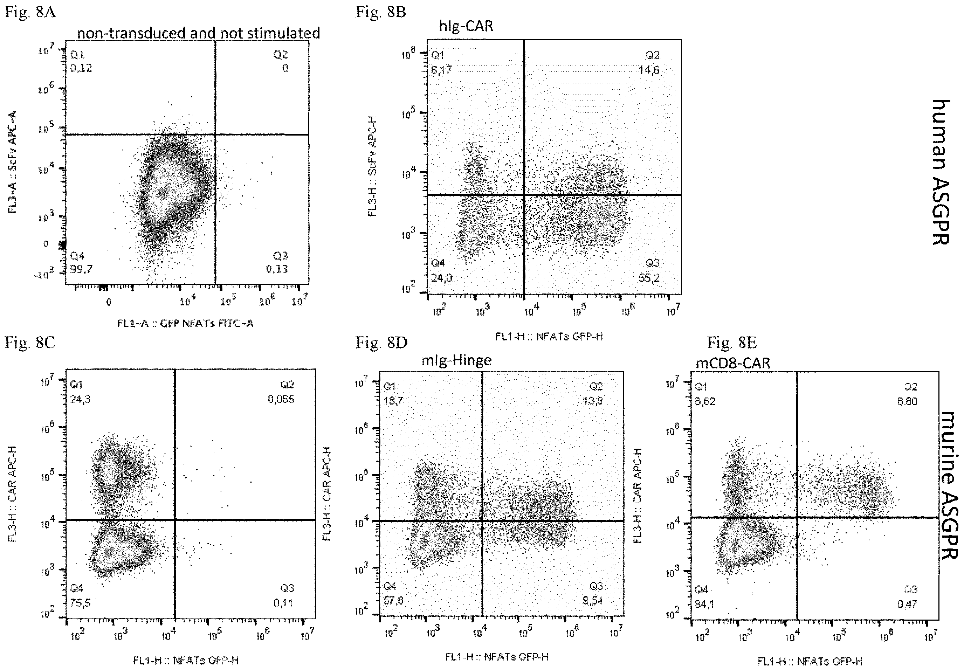

- Fig. 8A shows flow cytometry results for control Treg, and Fig. 8B-F show flow cytometry results for Treg expressing a CAR of the invention,

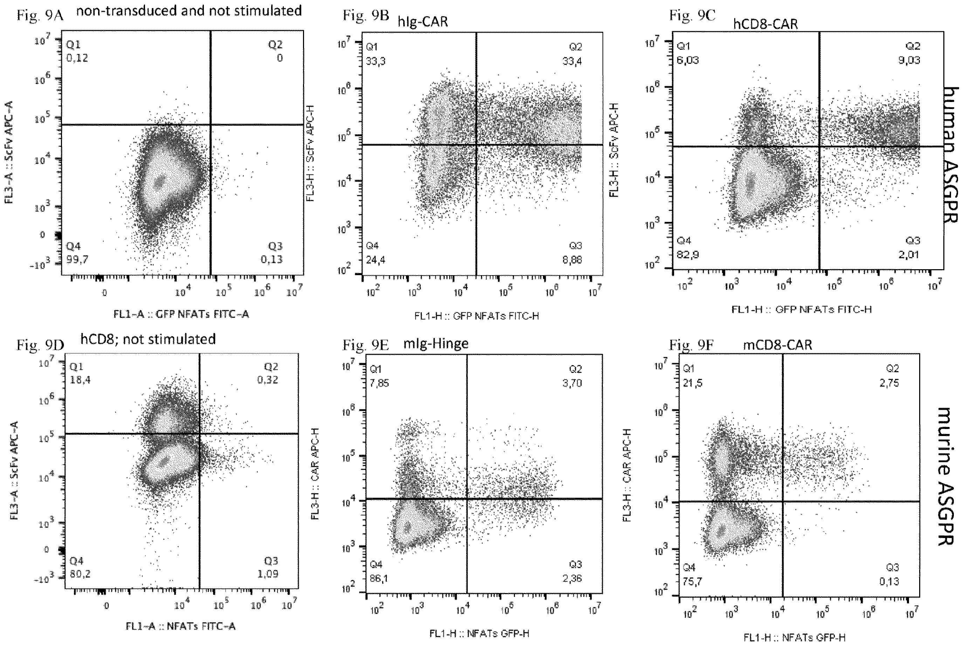

- Fig. 9A shows flow cytometry results for control Treg, and Fig. 9B-F show flow cytometry results for Treg expressing a CAR of the invention,

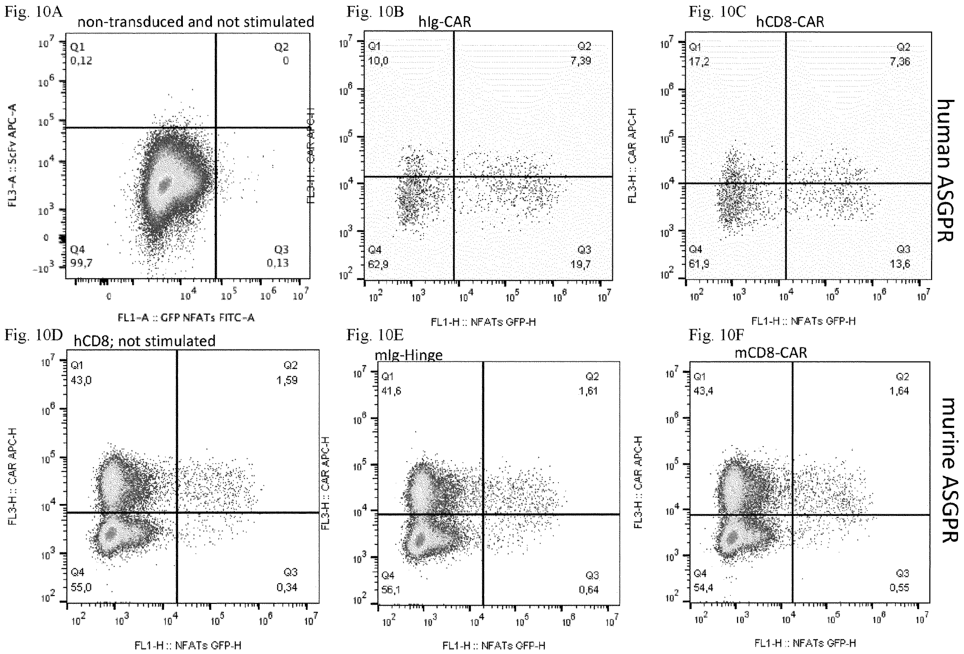

- Fig. 10A-F show flow cytometry results for Treg expressing a CAR of the invention with immune staining for the CAR and for the reporter Thy1.1, expressed as a portion of a fusion protein CAR-P2A-Thy1.1,

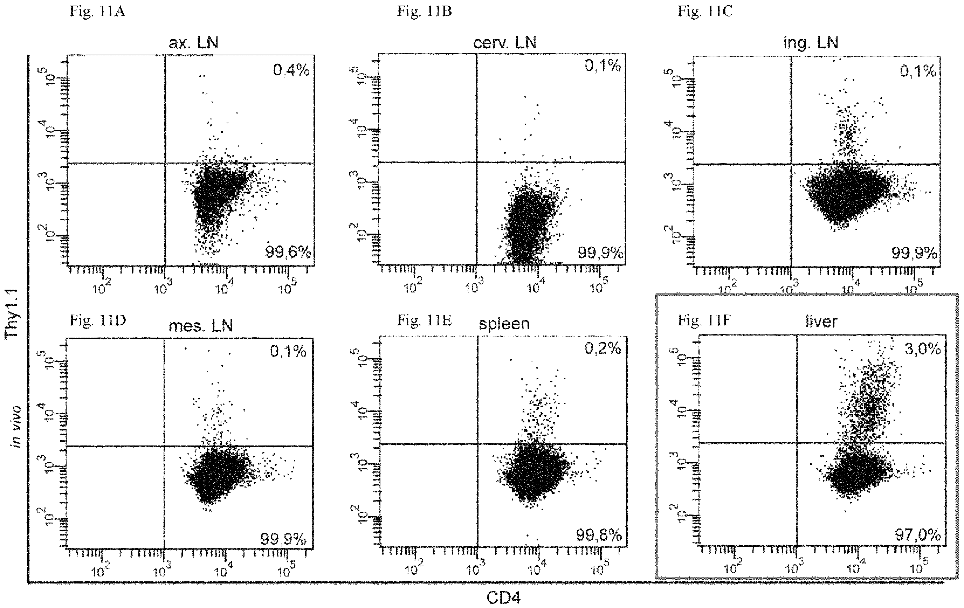

- Fig. 11A-F shows flow cytometry results of the distribution of Treg expressing an exemplary CAR after administration to a mouse,

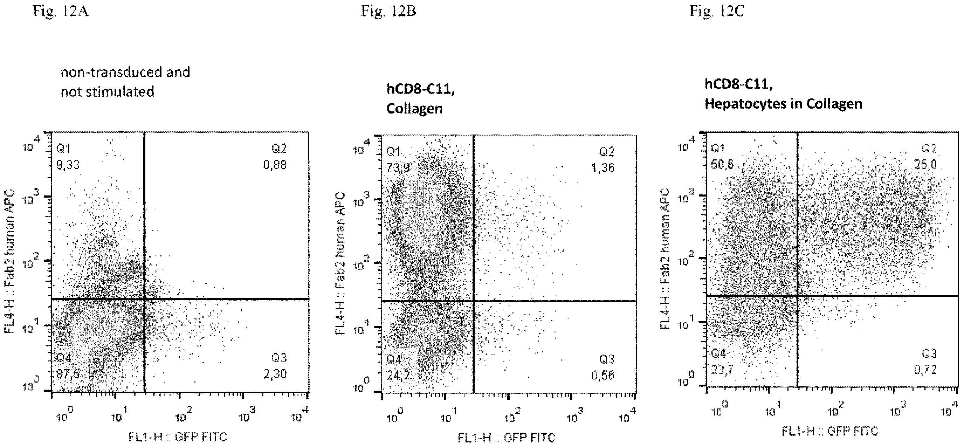

- Fig. 12A-C show flow cytometry results for the activation of Treg expressing a CAR of the invention specifically by presence of hepatocytes.

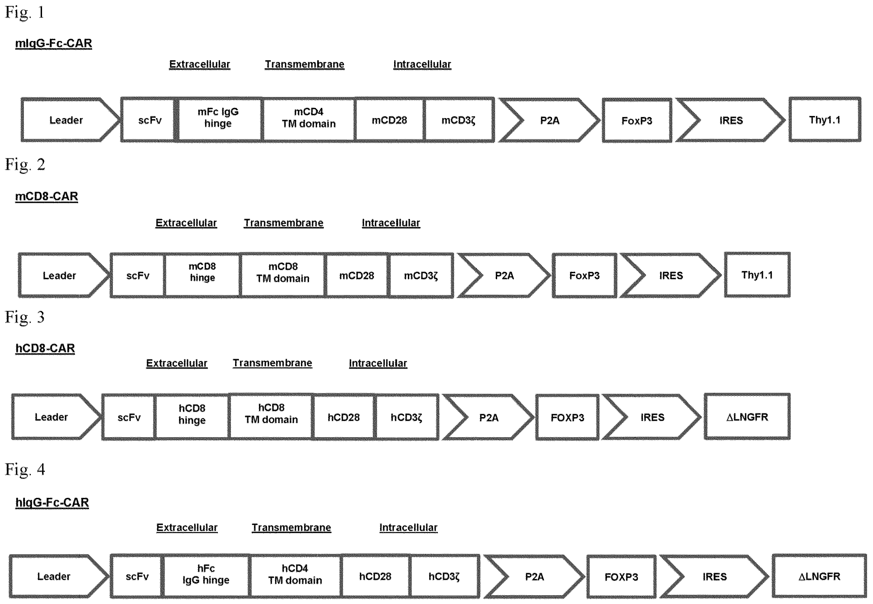

In the examples, the following CARs are used:
Signal peptide (SEQ ID NO: 1) - scFv-hinge of Ig G1 and IgG1 CH2 and IgG1 CH3 (SEQ ID NO: 5) - CD4 TM (SEQ ID NO: 9) - CD28 ICD (SEQ ID NO: 11) - CD3z ICD (SEQ ID NO: 12) - P2A (SEQ ID NO: 19) - FOXP3 (SEQ ID NO: 20), of murine origin and designated murine Ig-construct (mIg), which is schematically shown in Fig. 1,
Signal peptide (SEQ ID NO: 2) - scFv - CD8 hinge (SEQ ID NO: 6) - CD8a TM (SEQ ID NO: 10) - CD28 ICD (SEQ ID NO: 13) - CD3z ICD (SEQ ID NO: 14) - P2A - FOXP3 (SEQ ID NO: 21), of murine origin and designated murine CD8 construct (mCD8), which is schematically shown in Fig. 2,
Signal peptide (SEQ ID NO: 3) - scFv - CD8 hinge and CD8 TM (SEQ ID NO: 7) - CD28 ICD (SEQ ID NO: 15) - CD3z ICD (SEQ ID NO: 16) - P2A - FOXP3, of human origin and designated human CD8 construct (hCD8), which is schematically shown in Fig. 3,
Signal peptide (SEQ ID NO: 4) - scFv - deltaFc Ig (SEQ ID NO: 8) as hinge and transmembrane domain - CD28 ICD (SEQ ID NO: 17) - CD3z ICD (SEQ ID NO: 18), of human origin and designated human Ig construct (hIg), which is schematically shown in Fig. 4, with the specific scFv attached to the designation of the construct. Accordingly, the designations as of human origin or of murine origin generally only refer to the functional elements and domains except for the scFv, and independent the scFv.

The scFv sections have been generated by selection of scFv fragments contained in a phage display library according to affinity to immobilized ASGPR.

### Example 1: Expression of ASGPR-specific CAR and marker protein

Several CARs, containing one of the scFv of SEQ ID NO: 23 (G3), SEQ ID NO: 24 (C11) which includes a C-terminal His-tag, SEQ ID NO: 25 (E12) which includes a C-terminal His-tag, SEQ ID NO: 26 (C3), and SEQ ID NO: 27 (B10), were expressed in HEK cells that were transduced by nucleic acid constructs containing expression cassettes for one of the CARs. Each CAR had the structure as schematically shown in Fig. 1, encoding the CAR containing mIg as a hinge as a fusion protein with P2A and murine Foxp3 (mFoxp3), and with a separate internal ribosome entry site (IRES) encoding Thy 1.1.

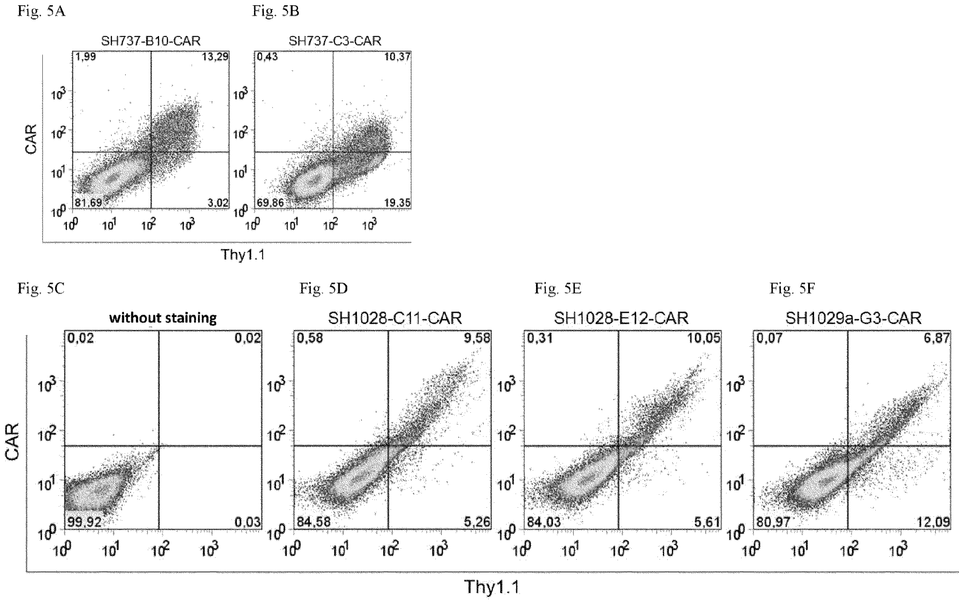

For analysis, cells that were transduced with a nucleic acid construct encoding one of the CARs were contacted with fluorescence-labelled anti-human-IgG-antibody for detecting the CAR and fluorescence-labelled anti-Thy1.1 antibody. Fig. 5 depicts the signals detected by flow cytometry, showing in comparison with Fig. 5A for non-transduced cells without staining as background control, and in Fig. 5B to 5F with immune-staining for the marker Thy 1.1 and immune-staining for the CAR. In relation to the cells detected in Fig. 5A as background, expressing both the marker and the CAR 9.58 % of cells for the scFv of SEQ ID NO: 24 (Fig. 5B), 10.05% of cells for the scFv of SEQ ID NO: 25 (Fig. 5C), 6.87% of cells for the scFv of SEQ ID NO: 26 (Fig. 5D), 13.29% of cells for the scFv of SEQ ID NO: 27 (Fig. 5E), 10.37% of cells for the scFv of SEQ ID NO: 26 (Fig. 5F). This shows that each of the CAR molecules is expressed and that Thy 1.1 from the IRES is expressed at an approximately equimolar ratio. This shows that the CAR for each of the scFv variants is expressed and that the nucleic acid construct is suitable for co-expressing the marker protein under the control of an IRES.

### Example 2: Activation of T cells expressing a CAR having the scFv portion of SEQ ID NO: 24 (C11) by ASGPR

CAR molecules that had the same scFv portion of SEQ ID NO: 24 (C11) were expressed in the reporter cell line from a nucleic acid construct that was transduced into the cells. The CARs had one of the structures of the CAR containing a hIg hinge (schematically shown in Fig. 4), the structure of the CAR containing a hCD8 hinge (schematically shown in Fig.3), the structure of the CAR containing a mIg hinge (schematically shown in Fig. 1), and with the structure of the CAR containing a mCD8 hinge (schematically shown in Fig. 2).

Fig. 6A shows flow cytometry results for non-transduced reporter cells in the absence of stimulation by ASGPR, indicating a background of 99.7% cells in quadrant 4 (Q4). Fig. 6D shows flow cytometry results for reporter cells that were transduced with a nucleic acid construct encoding the CAR in the absence of stimulation, indicating 55.2% of non-expressing cells in Q4 and 42.7% cells expressing the CAR (Q1, quadrant 1), as detected by the anti-human IgG-antibody that is specific for the CAR. Background is indicated at 1.00% (quadrant 2, Q2) and 1.02% (quadrant 3, Q3) for GFP expression.

These reporter cells were activated by contacting with ASGPR protein of the human amino acid sequence (Fig. 6B and 6C) or with ASGPR of the murine amino acid sequence (Fig. 6D, Fig. 6E and Fig. 6F) and analyzed by flow cytometry for presence of the CAR by labelled anti-human IgG-antibody and analyzes for activation by detecting the GFP signal as this is induced by the T cell activation via NFAT.

For activation by human ASGPR, Fig. 6B indicates an activation of 1.82% in Q2 for cells expressing the CAR and showing activation as measured by the GFP signal. Fig. 6C for human ASGPR indicates an activation of 1.31% in Q2 and of 11.6% in Q3, in total 12.91%, for CAR-expressing cells as measured by the GFP signal. In view of the controls without activation of Fig. 6A and Fig. 6D showing low background for non-transduced cells and for CAR-expressing cells, activation above background is indicated by cells in one of Q2 and Q3. These results show that the CAR having the scFv of SEQ ID NO: 24 both in the embodiment of the hinge being the hIg hinge and in the embodiment having the CD8 hinge recognizes human ASGPR and results in activation of the T cells.

Fig. 6E and Fig. 6F shows flow cytometry results for activation by murine ASGPR, in Fig. 6E indicating 3,41% (Q2) and 9.86% (Q3) activated cells, in total 13.27% for the embodiment of the hinge being murine Ig hinge, and in Fig. 6F indicating 4.04% (Q2) and 5.40% (Q3) activated cells, in total 9.44% for the embodiment of the hinge being murine CD8 hinge. These results show that the CAR having the scFv of SEQ ID NO: 24 both in the embodiment of the hinge being the murine hIg hinge and in the embodiment having the murine CD8 hinge recognizes murine ASGPR and results in activation of the T cells.

Further, the results show that this scFv is specific for both human and murine ASGPR and that a CAR containing this scFv can be used both in humans and in rodents, especially in mice and rats.

### Example 3: Expression of CAR having the scFv portion of SEQ ID NO: 25 (E12)

CAR molecules that had the same scFv portion of SEQ ID NO: 25 (E12) were expressed in reporter cells from a nucleic acid construct that was transduced into the cells. The CARs had one of the structures of the structure of CAR containing a hIg hinge (schematically shown in Fig. 4), the structure of hCD8 (schematically shown in Fig.3), the structure of the CAR containing a mIg hinge (schematically shown in Fig. 1), and with the structure of CAR containing a mCD8 hinge (schematically shown in Fig. 2).

The cells were activated by contacting with ASGPR protein of the human amino acid sequence (Fig. 7B and 7C) or with ASGPR of the murine amino acid sequence (Fig. 7E and Fig. 7F) and analyzed by flow cytometry.

Fig. 7A shows flow cytometry results for non-transduced reporter cells in the absence of stimulation by ASGPR, indicating a background of 99.7% cells in quadrant 4 (Q4). Fig. 7D shows flow cytometry results for reporter cells that were transduced with a nucleic acid construct encoding the CAR in the absence of stimulation, indicating 56.5% of non-expressing cells in Q4 and 43.1% cells expressing the CAR (Q1, quadrant 1), as detected by the anti-human IgG-antibody that is specific for the CAR. Background is indicated at 0.27% (quadrant 2, Q2) and 0.13% (quadrant 3, Q3) for GFP expression.

For activation by human ASGPR, Fig. 7B and Fig. 7C indicate only background for CAR-expressing cells as measured by the GFP signal, indicating this scFv is not activated by human ASGPR, both in the embodiment of the hinge being the hIg hinge and in the embodiment having the CD8 hinge.

Fig. 7E and Fig. 7F shows flow cytometry results for activation by murine ASGPR, in Fig. 7E indicating 14.3% (Q2) and 7.68% (Q3) activated cells, in total 21.98% for the embodiment of the hinge being murine Ig hinge, and in Fig. 7F indicating 7.37% (Q2) and 9.13% (Q3) activated cells, in total 16.5% for the embodiment of the hinge being murine CD8 hinge. These results show that the CAR having the scFv of SEQ ID NO: 25 both in the embodiment of the hinge being the murine hIg hinge and in the embodiment having the murine CD8 hinge recognizes murine ASGPR and results in activation of the T cells.

The results show that in comparison to transduced cells that express the CAR but were not stimulated due to absence of ASGPR (Fig. 7D), each of the CARs were activated by presence of murine ASGPR but not by human ASGPR. The results show that a CAR having this scFv is highly specific for murine ASGPR and that a CAR containing this scFv can be used in rodents, especially in mice and rats.

### Example 4: Expression of CAR having the scFv portion of SEQ ID NO: 23 (G3)

CAR molecules that had the same scFv portion of SEQ ID NO: 23 (G3) were expressed in reporter cells from a nucleic acid construct that was transduced into the cells. The CARs had one of the structures of the structure of CAR containing a hIg hinge (schematically shown in Fig. 4), the structure of CAR containing a mIg hinge (schematically shown in Fig. 1), and with the structure of CAR containing a mCD8 hinge (schematically shown in Fig. 2).

Fig. 8A shows flow cytometry results for non-transduced reporter cells in the absence of stimulation by ASGPR, indicating a background of 99.7% cells in quadrant 4 (Q4). Fig. 8D shows flow cytometry results for reporter cells that were transduced with a nucleic acid construct encoding the CAR in the absence of stimulation, indicating 75.5% of non-expressing cells in Q4 and 24.3% cells expressing the CAR (Q1, quadrant 1), as detected by the anti-human IgG-antibody that is specific for the CAR. Background is indicated at 0.065% (quadrant 2, Q2) and 0.11% (quadrant 3, Q3) for activation as indicated by GFP expression.

The cells were activated by contacting with ASGPR protein of the human amino acid sequence (Fig. 8B) or with ASGPR of the murine amino acid sequence (Fig. 8D and Fig. 8E) and analyzed by flow cytometry.

For activation by human ASGPR, Fig. 8B indicates an activation of 14.6% in Q2 and of 55.2% in Q3, in total 69.8%, for CAR-expressing cells as measured by the GFP signal. In view of the controls without activation of Fig. 6A and Fig. 6D showing low background for non-transduced cells and for CAR-expressing cells, activation above background is indicated by cells in one of Q2 and Q3. These results show that the CAR having the scFv of SEQ ID NO: 23 both in the embodiment of the hinge being the hIg hinge and in the embodiment having the CD8 hinge recognizes human ASGPR and results in activation of the T cells.

Fig. 8D and Fig. 8E shows flow cytometry results for activation by murine ASGPR, in Fig. 8D indicating 13.9% (Q2) and 9.54% (Q3) activated cells, in total 23.44% for the embodiment of the hinge being murine Ig hinge, and in Fig. 8E indicating 6.80% (Q2) and 0.47% (Q3) activated cells for the embodiment of the hinge being murine CD8 hinge. These results show that the CAR having the scFv of SEQ ID NO: 23 both in the embodiment of the hinge being the murine hIg hinge and in the embodiment having the murine CD8 hinge recognizes murine ASGPR and results in activation of the T cells.

The results show that in comparison to transduced cells that express the CAR but were not stimulated due to absence of ASGPR (Fig. 8C), each of the CARs were activated by presence of human or murine ASGPR. This shows that the activation of the T cell due to expression of the CAR according to the invention was stimulated by presence of ASGPR. Further, the results show that this scFv is specific for both human and murine ASGPR and that a CAR containing this scFv can be used both in humans and in rodents, especially in mice and rats.

### Example 5: Expression of CAR having the scFv portion of SEQ ID NO: 26 (C3)

CAR molecules that had the same scFv portion of SEQ ID NO: 26 (C3) were expressed in reporter cells from a nucleic acid construct that was transduced into the cells. The CARs had one of the structures of the structure of CAR containing a hIg hinge (schematically shown in Fig. 4), the structure of hCD8 (schematically shown in Fig.3), the structure of CAR containing a mIg hinge (schematically shown in Fig. 1), and with the structure of CAR containing a mCD8 hinge (schematically shown in Fig. 2).

Fig. 9A shows flow cytometry results for non-transduced reporter cells in the absence of stimulation by ASGPR, indicating a background of 99.7% cells in quadrant 4 (Q4). Fig. 9D shows flow cytometry results for reporter cells that were transduced with a nucleic acid construct encoding the CAR in the absence of stimulation, indicating 80.2% of non-expressing cells in Q4 and 18.4% cells expressing the CAR (Q1, quadrant 1), as detected by the anti-human IgG-antibody that is specific for the CAR. Background is indicated at 0.32% (quadrant 2, Q2) and 1.09 % (quadrant 3, Q3) for activation as indicated by GFP expression.

The cells were activated by contacting with ASGPR protein of the human amino acid sequence (Fig. 9B and 9C) or with ASGPR of the murine amino acid sequence (Fig. 9E and Fig. 9F) and analyzed by flow cytometry as described above.

For activation by human ASGPR, Fig. 9B indicates an activation of 33.4% in Q2 and 8.68 in Q3, in total 42.08% for cells expressing the CAR and showing activation as measured by the GFP signal. Fig. 9C for human ASGPR indicates an activation of 9.03% in Q2 and of 2.01% in Q3, in total 11.04%, for CAR-expressing cells as measured by the GFP signal. In view of the controls without activation of Fig. 9A showing low background for non-transduced cells and and Fig. 9D for CAR-expressing cells, activation above background is indicated by cells in one of Q2 and Q3. These results show that the CAR having the scFv of SEQ ID NO: 26 both in the embodiment of the hinge being the hIg hinge and in the embodiment having the CD8 hinge recognizes human ASGPR and results in activation of the T cells.

Fig. 9E and Fig. 9F shows flow cytometry results for activation by murine ASGPR, in Fig. 9E indicating 3.70% (Q2) and 2.36% (Q3), in total 6.06% activated cells for the embodiment of the hinge being murine Ig hinge, and in Fig. 9F indicating 2.75% (Q2) and 0.13% (Q3) activated cells, in total 2.88% for the embodiment of the hinge being murine CD8 hinge.

These results show that the CAR having the scFv of SEQ ID NO: 26 both in the embodiment of the hinge being the murine hIg hinge and in the embodiment having the murine CD8 hinge recognizes murine ASGPR and results in activation of the T cells.

The results show that in comparison to transduced cells that were not stimulated due to absence of ASGPR (Fig. 9D), each of the CARs were activated by presence of human or murine ASGPR. This shows that the activation of the reporter cells due to expression of the CAR according to the invention was stimulated by presence of ASGPR. Further, the results show that this scFv is specific for both human and murine ASGPR and that a CAR containing this scFv can be used both in humans and in rodents, especially in mice and rats.

### Example 6: Expression of CAR having the scFv portion of SEQ ID NO: 27 (B10)

CAR molecules that had the same scFv portion of SEQ ID NO: 27 (B10) were expressed in reporter cells from a nucleic acid construct that was transduced into the cells. The CARs had one of the structures of the structure of CAR containing a hIg hinge (schematically shown in Fig. 4), the structure of CAR containing a hCD8 hinge (schematically shown in Fig.3), the structure of CAR containing a mIg hinge (schematically shown in Fig. 1), and with the structure of CAR containing a mCD8 hinge (schematically shown in Fig. 2).

Fig. 10A shows flow cytometry results for non-transduced reporter cells in the absence of stimulation by ASGPR, indicating a background of 99.7% cells in quadrant 4 (Q4). Fig. 9D shows flow cytometry results for reporter cells that were transduced with a nucleic acid construct encoding the CAR in the absence of stimulation, indicating 55.0% of non-expressing cells in Q4 and 43.0% cells expressing the CAR (Q1, quadrant 1), as detected by the anti-human IgG-antibody that is specific for the CAR. Background for activation is indicated at 1.59% (quadrant 2, Q2) and 0.34 % (quadrant 3, Q3) as indicated by GFP expression.

The cells were activated by contacting with ASGPR protein of the human amino acid sequence (Fig. 10B and 10C) or with ASGPR of the murine amino acid sequence (Fig. Fig. 10E and Fig. 10F) and analyzed by flow cytometry for presence of the scFv portion.

For activation by human ASGPR, Fig. 10B indicates an activation of 7.39% in Q2 and 19.7 in Q3, in total 27.09% for cells expressing the CAR and showing activation as measured by the GFP signal. Fig. 10C for human ASGPR indicates an activation of 7.36% in Q2 and of 13.6% in Q3, in total 20.96%, for CAR-expressing cells as measured by the GFP signal. In view of the controls without activation of Fig. 10A showing low background for transduced cells and and Fig. 10D for CAR-expressing cells, activation above background is indicated by cells in one of Q2 and Q3. These results show that the CAR having the scFv of SEQ ID NO: 27 both in the embodiment of the hinge being the hIg hinge and in the embodiment having the CD8 hinge recognizes human ASGPR and results in activation of the T cells.

Fig. 10E and Fig. 9F shows flow cytometry results for activation by murine ASGPR, in Fig. 10E indicating 1.61% (Q2) and 0.64% (Q3), in total 2.25% activated cells for the embodiment of the hinge being murine Ig hinge, and in Fig. 10F indicating 1.64% (Q2) and 0.55% (Q3) activated cells, in total 2.19% for the embodiment of the hinge being murine CD8 hinge. These results show that the CAR having the scFv of SEQ ID NO: 27 is essentially not activated by murine ASGPR.

The results show that the reporter cells expressing this CAR were activated by presence of human ASGPR. The results show that this scFv is highly specific for human ASGPR and that a cell expressing a CAR containing this scFv can be used for the treatment especially in humans.

### Example 7: In vivo activity of Tree expressing a CAR

As a representative of a CAR of the invention, a CAR containing the scFv of SEQ ID NO: 27 (C11) in a CAR containing a mCD8 hinge (schematically shown in Fig. 2) was expressed in Treg cells that were transduced with a nucleic acid construct that also expressed FOXP3 in a fusion protein with the CAR.

These Treg cells were injected as a single dose of 3 million cells into NOD/Ltj mice by i. v. injection. Five days later, the mice were sacrificed and the presence of Treg expressing the CAR was analyzed by flow cytometry using immune-detection of Thy 1.1 as a representative of the co-expressed CAR and of CD4 as an indicator of activation of suppressor activity. Fig. 11 shows representative flow cytometry results in samples of axillary lymph nodes (ax. LN, Fig. 11A), cervical lymph nodes (cerv. LN, Fig. 11B), inguinal lymph nodes (ing. LN, Fig. 11C), mesenteric lymph nodes (mes. LN, Fig. 11D), spleen (Fig. 11E), and liver (Fig. 11F). In liver tissue, approx. 3% of lymphocytes were ASGPR-specific Treg expressing the CAR of the invention, while the lymphocytes in the control organs only contained approx. 0.1 to 0.4% ASGPR-specific Treg expressing the CAR of the invention.

These results show that the Treg cells expressing the CAR preferentially migrated into the liver and that their suppressor activity was activated essentially in liver only.

### Example 8: In vitro activity of Treg expressing a CAR

As a representative of a CAR of the invention, a CAR containing the scFv of SEQ ID NO: 27 (C11) in a CAR containing a hCD8 hinge (schematically shown in Fig. 3) was used in an activation assay. In short, reporter cells were transduced to express the CAR of the invention and stimulated by adding purified human hepatocytes.

Analysis was by flow cytometry with immune-staining using anti-humanCD8-antibody for detection of expression of the CAR and measuring GFP expression for detection of activation of suppressor activity. Fig. 12A for comparison shows untransduced cells without stimulation. Fig 12B for comparison shows the cells when exposed to collagen only, and Fig. 12C shows the cells when exposed to collagen and hepatocytes.

This result shows that Treg cells expressing the CAR and preferably also FOXP3 in a collagen-containing environment were activated for suppressor activity specifically by presence of hepatocytes.

The results also show that the specificity of the CAR is determined by its scFv and also show that expression of the CAR in the presence of ASGPR results in the activation of the suppressor activity of Treg.

## Claims

1. Liver-specific CAR containing from N-terminus to C-terminus an scFv portion, a hinge, a transmembrane domain and at least one signaling domain, wherein the scFv portion is specific for the asialoglycoprotein receptor (ASGPR) and wherein the scFv portion contains a pair of CDR3 selected from
- amino acids No. 97..113 of SEQ ID NO: 28 and amino acids No. 89..97 of SEQ ID NO: 29, or
- amino acids No. 99..109 of SEQ ID NO: 30 and amino acids No. 91..101 of SEQ ID NO: 31,
- or amino acids No. 97..108 of SEQ ID NO: 32 and amino acids No. 92..100 of SEQ ID NO: 33,
- or amino acids No. 97..108 of SEQ ID NO: 34 and amino acids No. 92..100 of SEQ ID NO: 35,
- or amino acids No. 97..107 of SEQ ID NO: 36 and amino acids No. 89..97 of SEQ ID NO: 37.

2. Liver-specific CAR according to one of the preceding claims, **characterized in that** in a regulatory T cell (Treg) the CAR is produced as a fusion protein with FOXP3 with a protease site arranged between the CAR and FOXP3.

3. Liver-specific CAR according to one of the preceding claims, **characterized in that** the CAR is encoded by a nucleic acid construct which downstream from the CAR encoding section contains a sequence encoding deltaLNGFR or CD20 epitope as a marker, under the control of an IRES.

4. Liver-specific CAR according to one of the preceding claims, **characterized in that** the scFv comprises a pair of variable chains selected from SEQ ID NO: 28 and SEQ ID NO: 29, SEQ ID NO: 30 and SEQ ID NO: 31, SEQ ID NO: 32 and SEQ ID NO: 33, SEQ ID NO: 34 and SEQ ID NO: 35, or SEQ ID NO: 36 and SEQ ID NO: 37.

5. Liver-specific CAR according to one of the preceding claims, **characterized in that** the CAR at the C-terminus of the scFv portion contains a hinge having an amino acid sequence of SEQ ID NO: 5 or of SEQ ID NO: 6.

6. Liver-specific CAR according to one of the preceding claims, **characterized in that** the CAR at the C-terminus of the scFv portion contains a hinge and a transmembrane domain having an amino acid sequence of SEQ ID NO: 7 or of SEQ ID NO: 8.

7. Liver-specific CAR according to one of the preceding claims for use in the treatment of an autoimmune disease directed against liver tissue, or for use in the treatment of inflammatory liver disorder.

8. Regulatory T cell expressing a CAR, **characterized in that** the regulatory T cell is genetically manipulated to express a CAR according to one of the preceding claims.

9. Regulatory T cell according to claim 8 for use in the treatment of an autoimmune disease directed against liver tissue, or for use in the treatment of inflammatory liver disorder in a patient, wherein the T cell is immunologically compatible with the patient.

10. Regulatory T cell according to claim 8 or 9 for use in the treatment of an autoimmune disease directed against liver tissue, or for use in the treatment of inflammatory liver disorder in a patient, wherein the T cell is an autologous cell originating from the patient to be treated or the T cell is derived from an autologous cell originating from the patient to be treated.

11. Regulatory T cell according to one of claims 8 to 10, **characterized in that** the T cell in the presence of liver tissue has immune suppressive activity.

12. In vitro process for producing a regulatory T cell according to one of claims 8 to 11, **characterized by** introducing into a T cell a nucleic acid construct encoding a CAR according to one of claims 1 to 7.
